# EUROPÄISCHE PATENTSCHRIFT

(11) **EP 3 403 634 B1**
(45) Veröffentlichungstag und Bekanntmachung des Hinweises auf die Patenterteilung: **31.08.2022**
(21) Anmeldenummer: 18168554.6
(22) Anmeldetag: 20.04.2018
(51) Int. Cl.: A61F 13/62, A44B 18/00, D04H 1/54, D04H 1/559, D04H 11/08, D04H 3/14

(54) **SCHLAUFENMATERIAL**
LOOP MATERIAL
MATI?RE BOUCLÉE

(30) Priorität: 04.05.2017 DE 102017109592
(43) Veröffentlichungstag der Anmeldung: 21.11.2018
(73) Patentinhaber: Lohmann-koester GmbH & Co. KG, 96146 Altendorf (DE)
(72) Erfinder: Drozdziok-Weinhardt, Magdalena, 96146 Altendorf (DE)
(74) Vertreter: Reuther, Martin

(56) Entgegenhaltungen:
- EP-A1- 1 279 348
- WO-A1-99/14046
- DE-A1-102004 053 469
- US-A1- 2007 178 273
- US-A1- 2008 260 989

## Beschreibung

Die Erfindung betrifft ein mehrlagiges Schlaufenmaterial, welches wenigstens eine Trägerlage und eine von der Trägerlage getragene Schlaufenlage umfasst, wobei sowohl die Trägerlage als auch die Schlaufenlage ein NonWoven umfassen und das NonWoven der Trägerlage über eine Prägung verfestigt ist.

Derartiges Schlaufenmaterial ist beispielsweise aus der EP 1 093 340 A1, der EP 1 525 345 B1, der EP 959 854 B1, der WO 2006/008662 A2, der EP 2 636 782 A1 oder der EP 2 137 346 A1 hinlänglich bekannt, wobei beispielsweise die EP 2 137 346 A1 auch ein alternatives Schlaufenmaterial beschreibt, welches auch in der EP 1 023 173 A1 oder der EP 1 863 364 A1 beispielhaft beschrieben ist und eine Trägerlage, die eine Folie aufweist und die Schlaufenlage trägt, umfasst. Während das Schlaufenmaterial, bei welchem sowohl die Trägerlage als auch die Schlaufenlage ein NonWoven umfassen, im Stand der Technik als verhältnismäßig kostengünstig und sehr flexibel gegenüber dem Schlaufenmaterial, bei welchem die Trägerlage als Folie ausgebildet ist, beschrieben ist, erscheint das Schlaufenmaterial mit einer Folie als Trägerlage gegenüber dem Schlaufenmaterial, bei welchem sowohl die Trägerlage als auch die Schlaufenlage ein NonWoven umfassen, besser zu verarbeiten, da dieses Material beispielsweise sehr effektiv über Vakuum während der Fertigung positioniert bzw. geführt werden kann.

Hierbei versteht es sich, dass derartige Schlaufenmaterialien durchaus zu verschiedensten Zwecken genutzt werden können. Eine der häufigsten Einsatzwecke ist hierbei die Verwendung im Zusammenspiel mit absorbierenden Hygieneprodukten, wie beispielsweise Windeln, bzw. als weibliche Komponente eines zwei-komponentigen mechanischen Verschlusssystems. Insofern bedingt dieses insbesondere häufig, dass zwar verhältnismäßig hohe Haltekräfte verlangt werden, damit die absorbierenden Artikel sich nicht während des Gebrauches lösen, wobei jedoch an sich die Zahl der Öffnungsvorgänge sowie die maximale Dauer der Beanspruchung unter Umständen verhältnismäßig gering sein kann.

Es ist Aufgabe vorliegender Erfindung, ein Schlaufenmaterial bereitzustellen, welches einerseits hinsichtlich seiner Luftdurchlässigkeit in großen Grenzen anpassbar und dennoch hinsichtlich der Verhakungseigenschaften verhältnismäßig stabil ist und welches andererseits verhältnismäßig kostengünstig und flexibel ist.

Die Aufgabe der Erfindung wird durch ein Schlaufenmaterial mit den Merkmalen der unabhängigen Ansprüche gelöst. Weitere, ggf. auch unabhängig hiervon, vorteilhafte Ausgestaltungen finden sich in den Unteransprüchen sowie der nachfolgenden Beschreibung.

Ein mehrlagiges Schlaufenmaterial, welches wenigstens eine Trägerlage und eine von der Trägerlage getragene Schlaufenlage umfasst und bei welchem sowohl die Trägerlage als auch die Schlaufenlage ein NonWoven umfassen und das NonWoven der Trägerlage über eine Prägung verfestigt ist, ist einerseits hinsichtlich seiner Luftdurchlässigkeit in großen Grenzen anpassbar und dennoch hinsichtlich der Verhakungseigenschaften verhältnismäßig stabil und andererseits verhältnismäßig kostengünstig und verhältnismäßig flexibel, wenn sich das mehrlagige Schlaufenmaterial dadurch auszeichnet, dass das NonWoven der Trägerlage über eine Prägung mit einer Prägungsflächendichte zwischen 10% und 95% verfestigt ist und die Schlaufenlage und die Trägerlage intermittierend mit einer Verbindungsflächendichte, welche unter der Prägungsflächendichte liegt, miteinander verbunden sind.

Hierbei wird von der Grunderkenntnis ausgegangen, dass an sich die Verbindungsflächendichte zwischen der Trägerlage und der Schlaufenlage ein bedeutendes Kriterium für die Luftdurchlässigkeit, welche per se durch die Verwendung von NonWoven in der Trägerlage und in der Schlaufenlage bereits gegeben ist, einerseits und die Flexibilität sowie die Verhakungseigenschaften andererseits darstellt. Durch eine Erhöhung der Verbindungsflächendichte lässt sich an sich die Luftdurchlässigkeit verringern, was jedoch zu Lasten der Flauschigkeit, der Flexibilität sowie der Verhakungsfähigkeit geht oder aber, beispielsweise bei der Verwendung von Klebstoffen, zu erhöhten Kosten führt. Dadurch, dass die Trägerlage über eine Prägung mit einer Prägungsflächendichte zwischen 10% und 95% verfestigt ist, kann jedoch die Luftdurchlässigkeit in weiten Grenzen über die Prägung der Trägerlage angepasst werden, während, solange die Verbindungsflächendichte unter der Prägungsflächendichte liegt, die Schlaufenlage mit einer für deren Eigenschaften optimalen Verbindungsflächendichte mit der Trägerlage verbunden sein kann. Dieses ermöglicht entsprechend stabile Verhakungseigenschaften, eine ausreichende Flauschigkeit der Schlaufenlage bzw. ausreichend niedrige Kosten bzw. eine ausreichende Flexibilität, auch wenn eine geringe Luftdurchlässigkeit gewünscht wird, da letztere letztlich über die Trägerlage und deren Prägungsflächendichte eingestellt bzw. angepasst werden kann.

An sich ist es bereits hinlänglich bekannt, dass durch ein Prägen die Luftdurchlässigkeit von NonWoven-Materialien beeinflusst werden kann, wie insbesondere die EP 2 137 346 A1 erläutert, da durch ein Prägen im Bereich der Prägung eine weitgehende Luftundurchlässigkeit des jeweiligen NonWovens erzielt werden kann. Durch eine geeignete Wahl der Prägungsflächendichte kann mithin die Luftdurchlässigkeit einer entsprechenden, NonWoven umfassenden Lage beeinflusst werden.

Bei einer Prägung werden im Bereich einer entsprechenden Prägestelle die Fasern verdichtet, was bis zu einer Filmbildung also einer filmartigen Ausbildung der ursprünglichen Faserfläche im Bereich der Prägestelle geschehen kann. Ein derartiges Prägen kann insbesondere durch ein gezieltes Aufbringen einer Prägekraft an der entsprechenden Prägestelle geschehen, wodurch dann an dieser Prägestelle eine entsprechende Verdichtung erfolgt. Ggf. kann der Prägevorgang noch ergänzend durch eine entsprechende thermische Belastung oder durch eine Ergänzung durch Klebstoff unterstützt werden. Durch die Verdichtung wird einerseits der Faserverbund an der Prägestelle gestärkt, was dann zu einer entsprechenden Verfestigung des NonWoven führt. Je nach konkreter Ausgestaltung des Prägevorgangs und der hieraus resultierenden Anordnung des entsprechenden Fasern zueinander bedingt die Prägung, neben einer entsprechenden Stabilisierung des Faserverbunds, eine Reduzierung der Lichtstreuung bzw. -brechung im Bereich der Prägestelle, was letztlich dann zu einer Absenkung der durch die Einzelfasern bedingten Opazität und mithin zu einer Erhöhung der Transparenz führt. Über den Anteil der verprägten Fläche kann dann also bei geeigneter Ausgestaltung der Fasern im Bereich der Prägestelle die Transparenz des jeweiligen Materials, also beispielsweise der Trägerlage oder auch der Schlaufenlage, beeinflusst werden.

Eine Prägung kann beispielsweise durch entsprechend ausgestaltete Prägewalzen, an denen im Bereich der Prägestellen entsprechend prägend wirksame Maßnahmen, wie beispielsweise Erhöhungen, vorgesehen sind, bereitgestellt werden.

In diesem Zusammenhang sei erläutert, dass der Begriff der Prägungsflächendichte das Verhältnis der geprägten Fläche zur Gesamtfläche der Trägerlage, oder einer sonstigen Lage bezeichnet. Dementsprechend bezeichnet die Verbindungsflächendichte den prozentualen Flächenanteil der Verbindung zwischen der Schlafenlage und der Trägerlage an der jeweiligen Gesamtfläche des mehrlagigen Schlaufenmaterials.

Vorzugsweise liegt die Prägungsflächendichte der Trägerlage über 15%, sodass die Luftdurchlässigkeit der Trägerlage nicht zu groß wird. Insbesondere ist es diesbezüglich von Vorteil, wenn die Prägungsflächendichte über 20% liegt. Auch kann dann gerade in Bereich der Prägung ein etwa aufgetragener Klebstoff nicht oder nur sehr wenig in das NonWoven der Trägerlage einsickern, so dass auch, falls ein Klebstoff beispielsweise zur Verbindung von Schlaufenlage und Trägerlage oder alternativ bzw. kumulativ zur Verbindung der Trägerlage an einer anderen Oberfläche genutzt wird, die Menge an benötigtem Klebstoff zur betriebssicheren Bereitstellung der entsprechend Verbindung minimiert werden kann. Je nach konkreter Ausgestaltung der jeweiligen Prägestellen, insbesondere in Abhängigkeit von dem dort zu findenden Verdichtungsgrad und/oder von der Dichte der jeweiligen Fasern zueinander, kann durch derartige Prägungsflächendichten die Transparenz der Trägerlage beeinflusst - und insbesondere auf einen Erhöhten Wert angehoben - werden. Dieses kann insbesondere auf die gesamte Transparenz des Schlaufenmaterials einen entscheidenden Einflusshaben, wenn die Faserdichte der Schlaufenlage verhältnismäßig gering ist, um bei möglichst niedrigem Materialeinsatz gute Verhakungseigenschaften zu bedingen, und die Schlaufenlage deswegen nur bedingt die Opazität nachteilig beeinflusst.

Durch eine Prägungsflächendichte unter 95% kann gewährleistet werden, dass die Trägerlage in sich zumindest derart verfestigt ist, dass sie, ggf. im Zusammenspiel mit der Verbindung zur Schlaufenlage und der hierdurch ergänzenden Stabilität, ihre Funktion als Trägerlage erfüllen kann, ohne die Beweglichkeit der Trägerlage bzw. des Schlaufenmaterials zu stark zu beeinträchtigen und eine ggf. erwünschte verbleibende Luftdurchlässigkeit ganz auszuschließen. Die Trägerlage lässt sich beweglicher ausgestalten, wenn die Prägungsflächendichte der Trägerlage unter 93%, vorzugsweise unter 91%, liegt, sodass die Trägerlage entsprechend besser ihre tragende Funktion erfüllen kann. Je nach konkret gewählter Prägungsflächendichte der Trägerlage kann immer noch eine gute Transparenz gewährleistet werden, wenn die Prägungsflächendichte der Trägerlage unter den vorsetehend genannten Obergrenzen liegt.

Insbesondere kann die Prägungsflächendichte zwischen 15 %, vorzugsweise 20 %, und 93 %, vorzugsweise 91 % liegen, um so die vorstehend genannten Vorteile kumuliert umsetzen zu können.

Vorzugsweise ist das NonWoven der Trägerlage ein Spinnvlies, welches per se insbesondere in sich bereits entsprechend mechanisch verfestigt ist (spunbond), sodass dieses NonWoven insbesondere im Zusammenspiel mit der vorstehend erläuterten Prägung in der Lage ist, der Trägerlage eine ausreichend tragende Funktionalität zu verleihen. Insbesondere kann das NonWoven der Trägerlage jedoch kumulativ bzw. alternativ hierzu thermisch stabilisiert (thermobond) sein. Vorzugsweise ist das Spinnvlies der Trägerlage thermisch gezogen oder heißluftgezogen (meltblown), was entsprechend preisgünstig eine hohe Festigkeit ermöglicht.

Es versteht sich, dass ggf. statt eines Spinnvlieses für das NonWoven der Trägerlage auch anders verfestigte NonWoven, wie beispielsweise wasserstrahlverfestigte NonWoven oder auch NonWoven, welche über chemische Maßnahmen oder ausschließlich Prägungen verfestigt wurden, kumulativ bzw. alternativ hierzu zur Anwendung kommen können, solange sichergestellt ist, dass die hierdurch bereitgestellte Trägerlage ihre Tragefunktion in ausreichendem Maße gewährleisten kann.

Vorzugsweise weist das NonWoven der Trägerlage eine Grammatur über 5 g/m² auf, was dazu führt, dass die Trägerlage entsprechend ausreichend stabil ausgebildet ist. Letzteres gilt insbesondere dann, wenn die Grammatur über 8 g/m² liegt.

Insbesondere aus Kostengründen aber auch hinsichtlich einer ausreichenden Flexibilität und Luftdurchlässigkeit ist es andererseits von Vorteil, wenn die Grammatur des NonWovens der Trägerlage unter 40 g/m² liegt, wobei es insbesondere von Vorteil ist, wenn die Grammatur unter 35 g/m² liegt, um die diesbezüglichen Vorteile in ausreichendem Maße nutzen zu können.

Insbesondere ist es dementsprechend von Vorteil, wenn das NonWoven der Trägerlage eine Grammatur zwischen 5 g/m² bzw. 8 g/m², einerseits und 40 g/m², insbesondere 35 g/m², andererseits aufweist.

Vorzugsweise ist das NonWoven der Schlaufenlage wasserstrahlverfestigt (spunlace), was, entsprechend der Hauptaufgabe der Schlaufenlage, gute Verhakungseigenschaften bzw. eine gute Flauschigkeit bedingt. Hierbei wird einerseits eine entsprechend hohe Weichheit bzw. Flauschigkeit angestrebt, was insbesondere durch eine möglichst geringe starre Fixierung einzelner Faserabschnitte zueinander bedingt sein kann. Durch eine geringe Fixierung, beispielsweise durch Schmelzpunkte oder ähnliches, können dann Längenreserven der einzelnen Fasern in dem jeweiligen NonWoven in z-Richtung, also senkrecht zur Flächenerstreckung der jeweiligen Lage bzw. des Schlaufenmaterials, verbleiben, die dann die Weichheit, die Flauschigkeit und entsprechend die Verhakungseigenschaften erhöhen.

Es versteht sich, dass ggf. auch andere Verfestigungsarten für das NonWoven der Schlaufenlage genutzt werden könne, solange das entsprechende NonWoven in ausreichendem Maße entsprechende Verhakungsmöglichkeiten belässt. So können beispielsweise eine chemische Verfestigung oder auch eine Ausgestaltung als Spinnfließ kumulativ bzw. alternativ dementsprechend zur Anwendung kommen. Selbiges gilt auch, ggf., für eine Prägung. Auch ist zu berücksichtigen, dass die Verbindung zwischen der Trägerlage und der Schlaufenlage entsprechend der Verbindungsflächendichte ggf. ebenfalls zu einer Verfestigung des NonWovens der Schlaufenlage beiträgt.

Vorzugsweise weist das NonWoven der Schlaufenlage eine Grammatur über 10 g/m² auf, was zu einer guten Stabilität der Schlaufenlage, insbesondere gegen ein unbeabsichtigtes Loslösen der Haken, dient. Dieses gilt umso mehr, wenn das NonWoven der Schlaufenlage eine Grammatur über 12 g/m² aufweist.

Insbesondere aus Kostengründen aber auch wegen der Haptik ist es von Vorteil, wenn das NonWoven der Schlaufenlage eine Grammatur unter 50 g/m² aufweist, wobei die entsprechenden Vorteile insbesondere dann auftreten, wenn die Grammatur des NonWovens der Schlaufenlage unter 45 g/m² liegt.

Dementsprechend ist es insbesondere von Vorteil, wenn die Grammatur des NonWovens der Schlaufenlage innerhalb der vorstehenden Grenzwerte, also zwischen 10 g/m², insbesondere 12 g/m², einerseits und 50 g/m², insbesondere 45 g/m², andererseits liegt.

Vorzugsweise wird bei dem Schlaufenmaterial, abgesehen von der Wasserverfestigung und der durch die Verbindung zur Trägerlage bedingte Verfestigung auf ergänzende, verfestigende Maßnahmen verzichtet, damit das Schlaufenmaterial so weit wie möglich seine Verhakungseigenschaften behält.

An sich kommt für die NonWoven sowohl der Schlaufenlage als auch der Trägerlage nahezu jedes Material, aus welchem ein NonWoven hergestellt werden kann, in Frage, solange die jeweils bestimmenden Eigenschaften, wie die Tragefunktion der Trägerlage und die Verhakungsfunktion bzw. Flauschigkeit der Schlaufenlage, unter Berücksichtigung der gewünschten Luftdurchlässigkeit, der Flexibilität des Materials und der Kosten umgesetzt werden können. Als besonders geeignet haben sich PP (Polypropylen), PE (Polyethylen) oder Mischungen hieraus erwiesen.

Insbesondere versteht es sich, dass das NonWoven der Trägerlage auch hinsichtlich seines Materials von dem NonWoven der Schlaufenlage abweichend gewählt werden kann, solange eine ausreichend stabile Verbindung mit einer Verbindungsflächendichte unter der Prägungsflächendichte der Trägerlage gewährleistet werden kann.

Unter besonderen Umständen kann es von Vorteil sein, wenn das Schlaufenmaterial luftundurchlässig ausgebildet ist, wenn eine Luftdurchlässigkeit an sich nicht zwingend erforderlich ist und eine verhältnismäßig hohe Prägungsflächendichte die Flexibilität des Schlaufenmaterials nicht in unerwünschtem Maße herabsetzt. Eine derartige Luftundurchlässigkeit kann bei gegebener Messmethodik bereits dadurch entstehen, dass trotz einer Prägungsflächendichte von 95% oder weniger, was an sich entsprechend eine fast 5% Einzelfasern aufweisende Flächendichte des NonWovens der Trägerlage bedingt, durch das Zusammenspiel mit der Schlaufenlage die Luftdurchlässigkeit insgesamt entsprechend herab gesetzt ist.

In diesem Zusammenhang sei erwähnt, dass die Luftdurchlässigkeit vorliegend insbesondere dadurch gemessen wird, dass die Strömungsgeschwindigkeit von Luft senkrecht durch eine Fläche des zu untersuchenden Materials bei festgelegtem Differenzdruck gemessen wird. Als Differenzdruck werden 200 Pa angesetzt. Der Prüfung sollte jeweils faltenfrei derart in ein entsprechendes Messgerät eingelegt werden, dass keine Luft über den Rand oder Kanten des Prüflings entweichen kann. Um reproduzierbare Ergebnisse zu erhalten, sollte das Prüfmaterial mindestens 4h bei 23 ± 2 °C und 50 ± 5 % relativer Luftfeuchtigkeit (Raumtemperatur) klimatisiert werden. Bei der Verwendung von Rollenware sind mindestens 24 h empfehlenswert. Je nach konkret verwendetem Messgerät empfehlen sich kreisrunde Prüflinge mit einem Durchmesser zwischen 80 cm² und 100 cm² oder rechteckige Prüflinge mit Abmessungen um die 90 x 90 mm. Bei Einspannen sollten Verwindungen oder Knitterflächen vermieden werden, was insbesondere durch eine leichte Spannung des Prüflings in der Prüflingsebene gewährleistet werden kann. Für eine Verbesserung der Reproduzierbarkeit und Vergleichbarkeit der Ergebnisse empfiehlt es sich die Prüflinge jeweils mit der Trägerlage zur Unterdr4uckseite anzuordnen. Ggf. kann die Strömungsgeschwindigkeit stufenweise bzw. schrittweise oder auch linear angepasst werden, bis sich der vorgegebene Differenzdruck eingestellt hat. Bei gegebenem Differenzdruck und gemessener Strömungsgeschwindigkeit kann dann aus der Fläche die Luftdurchlässigkeit ermittelt werden, wobei es sich empfiehlt wenigstens 10 Messungen an 10 unterschiedlichen Prüflingen eines Schlaufenmaterials durchzuführen, umk eine repräsentative Aussage aus Mittelwert und Standardabweichung zu erhalten.

Insbesondere kann die Luftdurchlässigkeit größer 50 l/m²/s liegen, was bereits über die Fläche des Schlaufenmaterials eine ausreichende Luftdurchlässigkeit zum Vermeiden von Staunässe und ähnlichem ermöglicht. Letzterer Vorteil gilt insbesondere dann, wenn die Luftdurchlässigkeit größer 80 l/m²/s ist.

Damit das Schlaufenmaterial bei einer Applikation mittels Vakuum ausreichend betriebssicher gehalten bzw. geführt werden kann, ist es von Vorteil, wenn die Luftdurchlässigkeit andererseits 1.000 l/m²/s oder weniger beträgt. Insbesondere ist es dementsprechend von Vorteil, wenn die Luftdurchlässigkeit kleiner 900 l/m²/s ist, sodass dementsprechend eine höhere Betriebssicherheit gewährleistet werden kann.

Vorzugsweise liegt die Luftdurchlässigkeit zwischen 0 l/m²/s, vorzugsweise 50 l/m²/s, insbesondere größer 80 l/m²/s, und 1.000 l/m²/s, vorzugsweise 900 l/m²/s, liegt.

An sich kommen als Verbindung zwischen der Trägerlage und der Schlaufenlage sämtliche Verbindungsarten in Frage, mit denen eine Verbindung zwischen zwei NonWoven-Lagen mit einer Verbindungsflächendichte, welche unterhalb der Prägeflächendichte liegt, in ausreichendem Maße betriebssicher gewährleistet werden kann.

Beispielsweise kann die Verbindung zwischen der Trägerlage und der Schlaufenlage eine Klebeverbindung umfassen, was letztlich betriebssicher und einfach bereitgestellt werden kann, da beispielsweise derartige Klebeverbindungen für Verbindungen zwischen einer Folie und einer NonWoven-Lage bereits bekannt sind. Hierbei hat sich herausgestellt, dass durch die mit einer Prägungsflächendichte zwischen 10% und 95% geprägte Trägerlage die Menge an Klebstoff, welche für eine Verbindung mit der Schlaufenlage gebraucht wird, sogar unerwartet niedrig ausfällt.

Die Verbindung zwischen der Trägerlage und der Schlaufenlage kann kumulativ bzw. alternativ hierzu insbesondere auch eine Prägung umfassen, was letztlich betriebssicher auf baulich einfache Weise bereitgestellt werden kann, wobei hierbei beachtet werden sollte, dass durch die Prägeverbindung zwischen der Trägerlage und der Schlaufenlage die Schlaufeneigenschaften der Schlaufenlage nicht zu stark nachteilig beeinflusst werden.

Insbesondere kann die Verbindungsflächendichte, wenn eine Prägung als Verbindung zwischen der Trägerlage und der Schlaufenlage zum Einsatz kommt, wenigstens 10 % unter der Prägungsflächendichte der Trägerlage liegen, so dass die Prägungsflächendichte der Trägerlage die Luftdurchlässigkeit und ggf. auch die Transparenz der Gesamtanordnung möglichst maßgeblich beeinflussen kann. Auch kann dann gewährleistet werden, dass durch die verbindende Prägung die Flauschigkeit, die Weichheit und mithin die Verhakungseigenschaften der Schlaufenlage möglichst wenig nachteilig beeinflusst werden.

Dementsprechend ist es insbesondere von Vorteil, wenn die Verbindungsflächendichte wenigstens 20 % unter der Prägungsflächendichte der Trägerlage liegt.

Auch ist es kumulativ bzw. alternativ hierzu denkbar, dass die Verbindung zwischen der Trägerlage und der Schlaufenlage eine Schweißverbindung umfasst. Je nach konkreter Art der Schweißverbindung, beispielsweise durch Ultraschallschweißen, kann sehr gezielt und bei minimaler Verbindungsdichte eine betriebssichere Verbindung zwischen dem NonWoven der Schlaufenlage und dem NonWoven der Trägerlage gewährleistet werden.

Die Verbindung zwischen der Trägerlage und der Schlaufenlage kann gitterförmig und linienförmig oder punktförmig ausgebildet sein. Derartige Verbindungsstrukturen lassen sich baulich einfach und mit ausreichend geringer Verbindungsflächendichte betriebssicher bereitstellen, zumal entsprechende maschinenbauliche Einrichtungen, mittels derer derartigen Verbindungen bereitgestellt werden können, hinlänglich aus dem Stand der Technik zur Verbindung von Flächenmaterialien bzw. von NonWoven untereinander bzw. mit sich selbst bekannt sind. Es versteht sich, dass gitterförmige, linienförmige oder punktförmige Verbindungen ggf. auch kombiniert oder überlagert werden können, solange die Verbindungsflächendichte innerhalb der vorgegebenen Grenzen bleibt. Auch versteht es sich, das Verbindung an sich in jeder Flächenverteilung zwischen der Schlaufenlage und der Trägerlage vorgesehen sein kann, solange die Verbindungsflächendichte im Vergleich zur Prägungsflächendichte ausreichend gering gewählt werden und eine über die Fläche ausreichend gleichförmige Verbindung gewährleistet werden kann.

Vorzugsweise liegt die Verbindungsflächendichte bei 5% und mehr, was eine entsprechend gute Verbindung zwischen der Trägerlage und der Schlaufenlage gewährleistet. Insbesondere kann die Verbindungsflächendichte über 10% liegen, was eine entsprechend größere Stabilität zwischen den beiden Lagen gewährleistet, solange die Verbindungsflächendichte unterhalb der Prägungsflächendichte der Trägerlage bleibt, um die Luftdurchlässigkeit durch das Schlaufenmaterial nicht zu beeinträchtigen.

Die Verbindungsflächendichte kann vorzugsweise unter 25% liegen, was einerseits in der Regel zu einer ausreichenden Luftdurchlässigkeit führt und andererseits die Schlaufen- und Verhakungseigenschaften der Schlaufenlage nicht zu stark beeinträchtigt. Dementsprechend vorteilhaft ist es, wenn die Verbindungsflächendichte unter 20% liegt, sodass die vorstehend genannten Vorteile entsprechend stärker umgesetzt sind.

Insbesondere liegt die Verbindungsflächendichte dementsprechend vorzugsweise zwischen 5%, insbesondere 10%, und 25%, insbesondere 20%.

Es versteht sich, dass die Merkmale der vorstehend bzw. in den Ansprüchen beschriebenen Lösungen gegebenenfalls auch kombiniert werden können, um die Vorteile entsprechend kumuliert umsetzen zu können.

Weitere Vorteile, Ziele und Eigenschaften vorliegender Erfindung werden anhand nachfolgender Beschreibung von Ausführungsbeispielen erläutert, die insbesondere auch in anliegender Zeichnung dargestellt sind. In der Zeichnung zeigen:
- Figur 1: einen schematischen Schnitt durch ein Schlaufenmaterial;
- Figur 2: eine schematische Darstellung der Flächenverteilung der Verbindung zwischen Trägerlage und Schlaufenlage sowie der Prägung der Trägerlage der Anordnung nach Fig. 1;
- Figur 3: eine schematische Darstellung der Flächenverteilung einer zur der Verbindung nach Fig. 2 alternativen Verbindung zwischen Trägerlage und Schlaufenlage sowie einer zu der Prägung nach Fig. 2 alternativen Prägung der Trägerlage;
- Figur 4: eine schematische Darstellung der Flächenverteilung einer zur der Verbindung nach Fign. 2 und 3 alternativen Verbindung zwischen Trägerlage und Schlaufenlage sowie einer zu der Prägung nach Fign. 2 und 3 alternativen Prägung der Trägerlage;
- Figur 5: eine schematische Darstellung der Flächenverteilung einer zur der Verbindung nach Fign. 2, 3 und 4 alternativen Verbindung zwischen Trägerlage und Schlaufenlage sowie einer zu der Prägung nach Fign. 2, 3 und 4 alternativen Prägung der Trägerlage;
- Figur 6: einen schematischen Schnitt durch ein zu dem Schlaufenmaterial nach Fig. 1 alternativen Schlaufenmaterial;
- Figur 7: einen schematischen Schnitt durch ein zu dem Schlaufenmaterial nach Fign. 1 und 6 alternativen Schlaufenmaterial; und
- Figur 8: einen schematischen Schnitt durch ein zu dem Schlaufenmaterial nach Fign. 1, 6 und 7 alternativen Schlaufenmaterial.

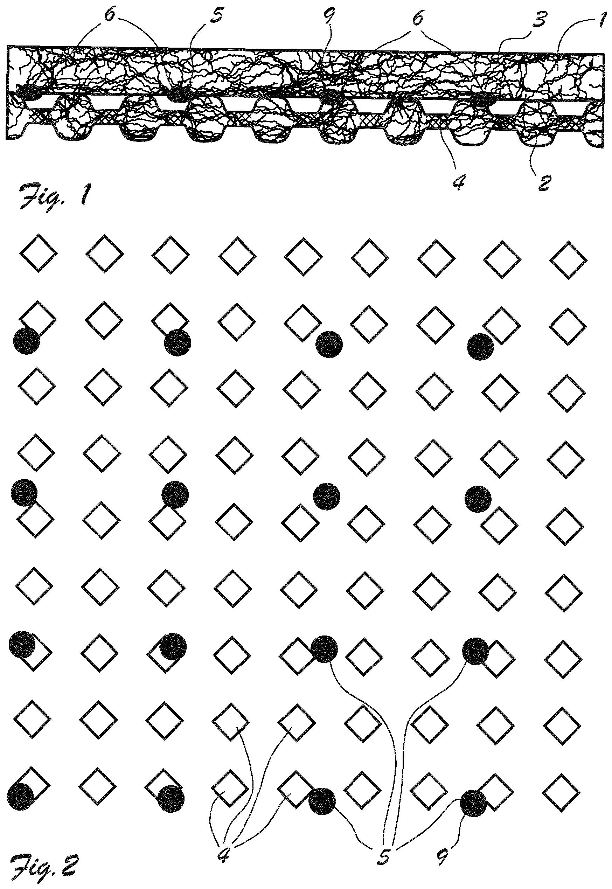

Das in Figuren 1 und 2 dargestellte Schlaufenmaterial 1 weist eine Trägerlage 2 und eine Schlaufenlage 3 auf, die über eine als Klebeverbindung 6 ausgebildete Verbindung 5 miteinander verbunden sind.

Die Trägerlage 2 ist über eine Prägung 4 verfestigt, wobei die Prägungsflächendichte dieses Ausführungsbeispiels bei ca. 30% liegt.

Die Klebeverbindung 6 umfasst einzelne Verbindungspunkte 9, wie insbesondere in Figur 2 ersichtlich, wobei die Verbindungsflächendichte dieser Verbindungspunkte 9 deutlich unter der Prägungsflächendichte der Prägung 4 liegt.

Die Schlaufenlage 3 ist bei diesem Ausführungsbeispiel aus wasserstrahlverfestigten Vlies bzw. NonWoven gebildet, wobei hier ggf. auch andere Verfestigungsarten zur Anwendung kommen. Die Grammatur liegt zwischen 15 g/m² und 40 g/m².

Die Trägerlage 2 ist aus einem Spinnvlies gebildet mit einer Grammatur zwischen zwischen 10 g/m² und 30 g/m² gebildet, wobei hier ggf. statt eines Spinnvlieses auch anders verfestigte Vliese zur Anwendung kommen können, solange die Stabilität der Gesamtanordnung gewährleistet werden kann.

Sowohl das NonWoven der Trägerlage 2 als auch das NonWoven der Schlaufenlage 3 sind bei diesem Ausführungsbeispiel aus PP gebildet. In alternativen Ausführungsformen können hier PE oder Mischungen davon zur Anwendung kommen.

Die Luftdurchlässigkeit des Schlaufenmaterials 1 nach diesem Ausführungsbeispiel liegt unter 800 l/m²/s, sodass das Schlaufenmaterial einfach mittels Vakuum gehandhabt werden kann.

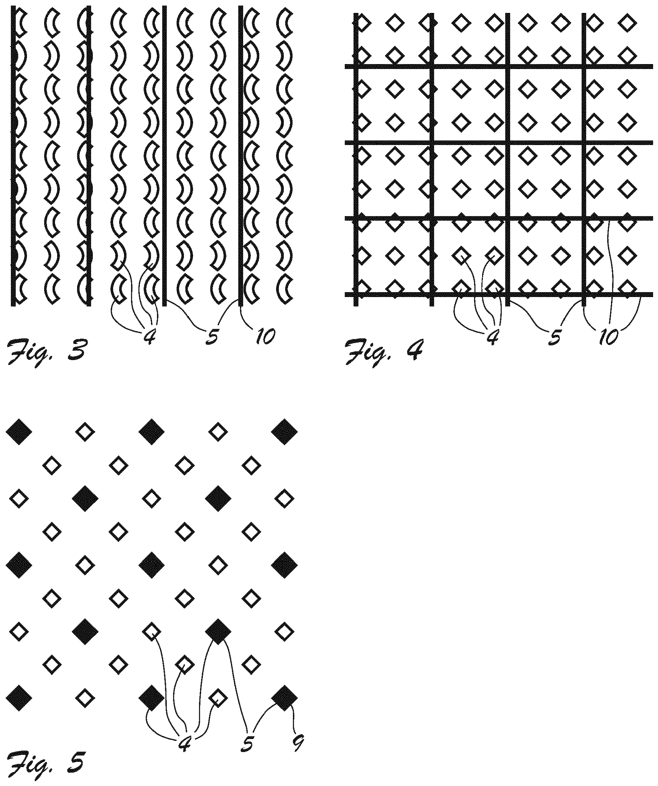

Das in Figur 3 schematisch dargestellte Ausführungsbeispiel entspricht im Wesentlichen dem Ausführungsbeispiel nach Figuren 1 und 2, unterscheidet sich jedoch durch die Prägung 4 der Trägerlage 2, welche insbesondere auch eine höhere Prägungsflächendichte, die bei diesem Ausführungsbeispiel bei ungefähr 36% liegt, aufweist.

Statt der Verbindungspunkte 9 weist die Anordnung nach Figur 3 Verbindungslinien 10 auf, die parallel zwischen der Trägerlage und der Schlaufenlage 3 verlaufen. Vorzugsweise verlaufen diese Verbindungslinien 10 in Maschinenrichtung, welche die Richtung beschreibt, entlang welcher die Trägerlage 2 und die Schlaufenlage 3 eine entsprechende Fertigungsmaschine, in welcher die Trägerlage 2 und die Schlaufenlage 3 verbunden werden, durchläuft. Dementsprechend brauchen die Werkzeuge, mittels welcher die Verbindungslinien 10 bereitgestellt werden, lediglich an Ort und Stelle verbleiben, während die zugehörigen Materialbahnen in Maschinenrichtung an ihnen vorbeilaufen.

Im Übrigen entsprechen die Parameter des Schlaufenmaterials 1 nach Figur 3 den Parametern des Schlaufenmaterials 1 nach Figuren 1 und 2, und können auch innerhalb der dort beschriebenen Grenzen variiert werden.

Das in Figur 4 dargestellte Schlaufenmaterial 1 entspricht bis auf die Verbindungslinien 10 dem Ausführungsbeispiel nach Figuren 1 und 2, wobei auch hier die Variationsmöglichkeit, welche anhand des Ausführungsbeispiels nach Figuren 1 und 2 beschrieben sind, genutzt werden können.

In Abweichung zu dem Ausführungsbeispiel nach Figur 3 weist das Schlaufenmaterial 1 nach Figur 4 eine gitterförmige Verbindung 5 zwischen der Trägerlage 2 und der Schlaufenlage 3 auf, welche durch entsprechend gekreuzte Verbindungslinien 10 bereitgestellt ist. Es versteht sich, dass in abweichenden Ausführungsformen die Verbindungslinien 10 nicht zwingend rechtwinklig bzw. in Bezug auf die Maschinenrichtung ausgerichtet verlaufen müssen. Letztlich sind hier äußerst viele Variationsmöglichkeiten vorhanden, solange diese bei ausreichend geringen Kosteneinsatz betriebssicher realisiert werden können. Auch hängt es auch von der Art der Verbindung 5 zwischen der Trägerlage 2 und der Schlaufenlage 3 ab, inwieweit Verbindungspunkte 9 oder Verbindungslinien 10 zur Anwendung kommen und in welchem Maße die flächige Ausgestaltungen der Verbindung 5 von Punkten oder in Maschinenrichtung verlaufenden Linien abweichen können. Quer zur Maschinenrichtung verlaufende Verbindungslinien 10 können beispielsweise durch Walzen oder ähnliches bereitgestellt werden.

Auch die Prägung 4 der Trägerlage 2 des Schlaufenmaterials 1, welches in Figur 5 exemplarisch dargestellt ist, entspricht der Prägung 4 der Ausführungsbeispiele nach Figuren 1, 2 und 4.

Auch bei dem in Figur 5 dargestellten Schlaufenmaterials 1 ist die Verbindung 5 durch Verbindungspunkte 9 realisiert, wobei die Verbindungspunkte 9 jeweils auf jedem zweiten Prägepunkt der Prägung 4 zu finden sind, was insbesondere für eine Klebeverbindung von Vorteil ist, da hier der Klebstoff betriebssicher auf der durch die Prägung verfestigten Oberfläche der Trägerlage 2 abgelegt werden kann und nicht in dem NonWoven versickert. Hierdurch kann die Menge an Klebstoff auf ein Minimum reduziert werden.

Auch bei dem in Figur 5 dargestellten Ausführungsbeispiel kommen im Übrigen Materialien und Variationen zur Anwendung, wie sie auch anhand des Ausführungsbeispiels nach Figuren 1 und 2 bereits erläutert wurde.

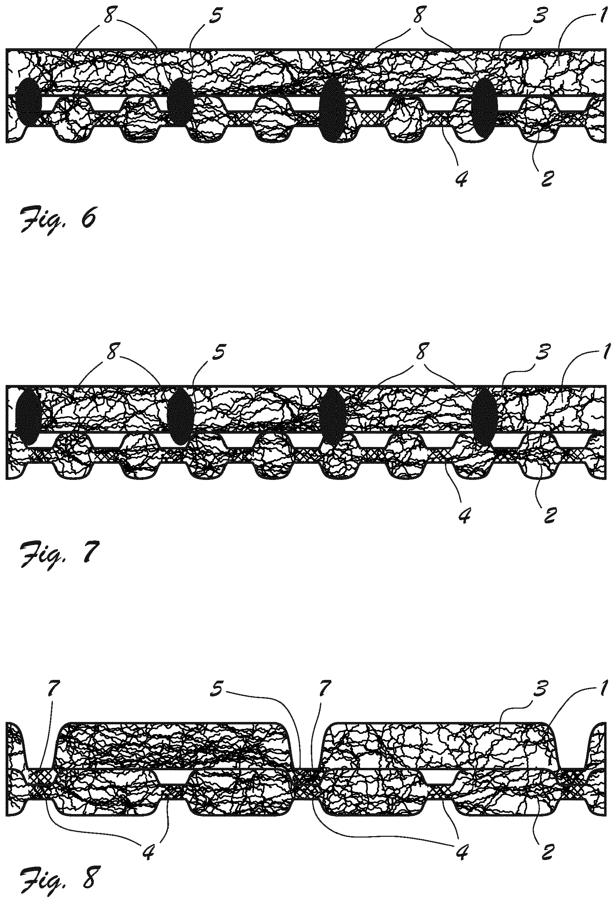

Auch eine verbindende Prägung 7 kann zur Anwendung kommen, wie dieses beispielhaft in Figur 8 dargestellt ist und beispielhaft zu einer Flächenverteilung, wie sie in Figur 5 dargestellt ist, führt, was sich als vorteilhaft erweist, da dann die verbindende Prägung 7 gleichzeitig auch als verfestigende Prägung 4 der Trägerlage 2 dienen kann.

Es versteht sich, dass ggf. die beiden Prägungen 4 und 7 nicht zwingend über ganzzahlige Perioden in räumlicher Beziehung stehen müssen. Hier können, ähnlich der in Figuren 1 bis 4 dargestellten Ausführungsbeispiele auch unterschiedliche Periodizitäten für die Prägung 4 und die Verbindung 5 zur Anwendung kommen.

Bei dem in Figuren 6 und 7 dargestellten Ausführungsbeispiel ist die Verbindung 5 jeweils durch Schweißverbindungen 8 bereitgestellt, die jedoch in unterschiedlicher Eindringtiefe innerhalb der Trägerlage 2 bzw. der Schlaufenlage zu finden sind.

Während das in Figur 6 dargestellte Ausführungsbeispiel durch eine thermische Belastung die Schweißverbindung 8 bereitstellt, sodass diese von der Trägerlage 2 ausgehend über die eingebrachte Wärme bis zu der Schlaufenlage 3 reichen, kommt bei den in Figur 7 dargestellten Ausführungsbeispiel Ultraschallschweißen für die Schweißverbindung 8 zur Anwendung, wobei der Ultraschall derart fokussiert ist, dass die Schweißverbindung 8 von Seiten der Schlaufenlage 3 eingebracht im Inneren des Schlaufenmaterials 1 ausgebildet wird.

Sämtliche Schlaufenmaterialien 1, die vorliegend dargestellt sind, bestehen aus PP, PE oder Mischungen davon und haben eine Luftdurchlässigkeit unter 800 l/m²/s.

### Bezugszeichenliste:

- 1: Schlaufenmaterial
- 2: Trägerlage
- 3: Schlaufenlage
- 4: Prägung der Trägerlage 2
- 5: Verbindung zwischen Trägerlage 2 und Schlaufenlage 3
- 6: Klebeverbindung
- 7: verbindende Prägung
- 8: Schweißverbindung
- 9: Verbindungspunkt
- 10: Verbindungslinie

## Patentansprüche

1. Mehrlagiges Schlaufenmaterial (1), welches wenigstens eine Trägerlage (2) und eine von der Trägerlage (2) getragene Schlaufenlage (3) umfasst, wobei sowohl die Trägerlage (2) als auch die Schlaufenlage (3) ein NonWoven umfassen und das NonWoven der Trägerlage (2) über eine Prägung (4) verfestigt ist, **dadurch gekennzeichnet, dass** das NonWoven der Trägerlage (2) über eine Prägung (4) mit einer Prägungsflächendichte zwischen 10 % und 95 % verfestigt ist und die Schlaufenlage (3) und die Trägerlage (2) intermittierend mit einer Verbindungsflächendichte, welche unter der Prägungsflächendichte liegt, miteinander verbunden sind.

2. Schlaufenmaterial (1) nach Anspruch 1, **dadurch gekennzeichnet, dass** die Prägungsflächendichte der Trägerlage (2) über 15 %, vorzugsweise über 20 %, und/oder unter 93 %, vorzugsweise unter 91 %, liegt.

3. Schlaufenmaterial (1) nach Anspruch 1 oder 2, **dadurch gekennzeichnet, dass** das NonWoven der Trägerlage (2) ein Spinnvlies ist.

4. Schlaufenmaterial (1) nach einem der Ansprüche 1 bis 3, **dadurch gekennzeichnet, dass** das NonWoven der Trägerlage (2) eine Grammatur zwischen 5 g/m² und 40 g/m², vorzugsweise über 8 g/m² und/oder unter 35 g/m², aufweist.

5. Schlaufenmaterial (1) nach einem der Ansprüche 1 bis 4, **dadurch gekennzeichnet, dass** das NonWoven der Schlaufenlage (3) wasserstrahlverfestigt ist.

6. Schlaufenmaterial (1) nach einem der Ansprüche 1 bis 5, **dadurch gekennzeichnet, dass** das NonWoven der Schlaufenlage (3) eine Grammatur zwischen 10 g/m² und 50 g/m², vorzugsweise über 12 g/m² und/oder unter 45 g/m², aufweist.

7. Schlaufenmaterial (1) nach einem der Ansprüche 1 bis 6, **dadurch gekennzeichnet, dass** das NonWoven PP, PE oder Mischungen hieraus umfasst.

8. Schlaufenmaterial (1) nach einem der Ansprüche 1 bis 7, **gekennzeichnet durch** eine Luftdurchlässigkeit zwischen 0 l/m²/s und 1.000 l/m²/s, vorzugsweise größer 50 l/m²/s, insbesondere größer 80 l/m²/s, und/oder kleiner 900 l/m²/s.

9. Schlaufenmaterial (1) nach einem der Ansprüche 1 bis 8, **dadurch gekennzeichnet, dass** die Verbindung (5) zwischen der Trägerlage (2) und der Schlaufenlage (3) eine Klebeverbindung (6), eine Prägung (7) und/oder eine Schweißverbindung (8) umfasst.

10. Schlaufenmaterial nach Anspruch 9, **dadurch gekennzeichnet, dass** die Verbindung (5) zwischen der Trägerlage (2) und der Schlaufenlage (3) ein Prägung (7) ist und die Verbindungsflächendichte wenigstens 10 %, vorzugsweise wenigstens 20 %, unter der Prägungsflächendichte der Trägerlage (2) liegt.

11. Schlaufenmaterial nach einem der Ansprüche 1 bis 10, **dadurch gekennzeichnet, dass** die Verbindung (5) zwischen der Trägerlage (2) und der Schlaufenlage (3) gitterförmig, linienförmig oder punktförmig ausgebildet ist.

12. Schlaufenmaterial nach einem der Ansprüche 1 bis 11, **dadurch gekennzeichnet, dass** die Verbindungsflächendichte zwischen 5 % und 25 %, vorzugsweise über 10 % und/oder unter 20 %, liegt.

## Claims

1. A multi-layered loop material (1), which comprises at least one support layer (2) and a loop layer (3) supported by the support layer (2), both the support layer (2) and the loop layer (3) comprising a non-woven, and the non-woven of the support layer (2) being compacted by means of a stamp (4), **characterized in that** the non-woven of the support layer (2) is compacted by means of a stamp (4) with a stamp density per unit area of between 10 % and 95 % and the loop layer (3) and the support layer (2) are intermittently connected to one another with a connection density per unit area, which is less than the stamp density per unit area.

2. The loop material (1) according to Claim 1, **characterized in that** the stamp density per unit area of the support layer (2) is greater than 15 %, preferably greater than 20 %, and/or below 93 %, preferably below 91 %.

3. The loop material (1) according to Claim 1 or 2, **characterized in that** the non-woven of the support layer (2) is a spunbonded fleece.

4. The loop material (1) according to one of Claims 1 to 3, **characterized in that** the non-woven of the support layer (2) has a grammage between 5 g/m² and 40 g/m², preferably above 8 g/m² and/or below 35 g/m².

5. The loop material (1) according to one of Claims 1 to 4, **characterized in that** the non-woven of the loop layer (3) is spunlaced.

6. The loop material (1) according to one of Claims 1 to 5, **characterized in that** the non-woven of the loop layer (3) has a grammage between 10 g/m² and 50 g/m², preferably above 12 g/m² and/or below 45 g/m².

7. The loop material (1) according to one of Claims 1 to 6, **characterized in that** the non-woven comprises PP, PE or mixtures thereof.

8. The loop material (1) according to one of Claims 1 to 7, **characterized by** an air permeability between 0 l/m²/s and 1,000 l/m²/s, preferably greater than 50 l/m²/s, particularly greater than 80 l/m²/s, and/or smaller than 900 l/m²/s.

9. The loop material (1) according to one of Claims 1 to 8, **characterized in that** the connection (5) between the support layer (2) and the loop layer (3) comprises an adhesively-bonded connection (6), a stamp (7) and/or a welded connection (8).

10. The loop material according to Claim 9, **characterized in that** the connection (5) between the support layer (2) and the loop layer (3) is a stamp (7) and the connection density per unit area is at least 10 %, preferably at least 20 %, below the stamp density per unit area of the support layer (2).

11. The loop material according to one of Claims 1 to 10, **characterized in that** the connection (5) between the support layer (2) and the loop layer (3) is constructed in a lattice-like, linear or punctiform manner.

12. The loop material according to one of Claims 1 to 11, **characterized in that** the connection density per unit area is between 5 % and 25 %, preferably above 10 % and/or below 20 %.

## Revendications

1. Matière bouclée multicouches (1), laquelle comprend au moins une couche de support (2) et une couche bouclée portée par la couche de support (2), aussi bien la couche de support (2) qu'également la couche bouclée (3) comprenant un non-tissé et le non-tissé de la couche de support (2) étant consolidé par un gaufrage (4), **caractérisée en ce que** le non-tissé de la couche de support (2) est consolidé par un gaufrage (4) faisant preuve d'une densité de surface de gaufrage comprise entre 10 % et 95 % et la couche bouclée (3) et la couche de support (2) étant assemblées l'une à l'autre par intermittence avec une densité de surface d'assemblage, laquelle est inférieure à la densité de surface de gaufrage.

2. Matière bouclée (1) selon la revendication 1, **caractérisée en ce que** la densité de surface de gaufrage de la couche de support (2) est supérieure à 15 %, de préférence supérieure à 20 %, et/ou inférieure à 93 %, de préférence inférieure à 91 %.

3. Matière bouclée (1) selon la revendication 1 ou 2, **caractérisée en ce que** le non-tissé de la couche de support (2) est un filé-lié.

4. Matière bouclée (1) selon l'une quelconque des revendications 1 à 3, **caractérisée en ce que** le non-tissé de la couche de support (2) fait preuve d'un grammage compris entre 5 g/m² et 40 g/m², de préférence supérieur à 8 g/m² et/ou inférieur à 35 g/m².

5. Matière bouclée (1) selon l'une quelconque des revendications 1 à 4, **caractérisée en ce que** le non-tissé de la couche bouclée (3) est consolidé au jet d'eau.

6. Matière bouclée (1) selon l'une quelconque des revendications 1 à 5, **caractérisée en ce que** le non-tissé de la couche bouclée (3) fait preuve d'un grammage compris entre 10 g/m² et 50 g/m², de préférence supérieur à 12 g/m² et/ou inférieur à 45 g/m².

7. Matière bouclée (1) selon l'une quelconque des revendications 1 à 6, **caractérisée en ce que** le non-tissé comprend du PP, du PE ou des mélanges de ces derniers.

8. Matière bouclée (1) selon l'une quelconque des revendications 1 à 7, **caractérisée par** une perméabilité à l'air comprise entre 0 l/m²/s et 1.000 l/m²/s, de préférence, supérieure à 50 l/m²/s, notamment supérieure à 80 l/m²/s, et/ou inférieure à 900 l/m²/s.

9. Matière bouclée (1) selon l'une quelconque des revendications 1 à 8, **caractérisée en ce que** l'assemblage (5) entre la couche de support (2) et la couche bouclée (3) comprend un assemblage par collage (6), un gaufrage (7) et/ou une soudure (8).

10. Matière bouclée selon la revendication 9, **caractérisée en ce que** l'assemblage (5) entre la couche de support (2) et la couche bouclée (3) est un gaufrage (7) et la densité de la surface d'assemblage est inférieure d'au moins 10 %, de préférence d'au moins 20 % à la densité de la surface de gaufrage de la couche de support (2).

11. Matière bouclée selon l'une quelconque des revendications 1 à 10, **caractérisée en ce que** l'assemblage (5) entre la couche de support (2) et la couche bouclée (3) est conçu en forme de grille, de forme linéaire ou de forme ponctuelle.

12. Matière bouclée selon l'une quelconque des revendications 1 à 11, **caractérisée en ce que** la densité de la surface d'assemblage est comprise entre 5 % et 25 %, de préférence supérieure à 10 % et/ou inférieure à 20 %.
